# FASCICULE DE BREVET EUROPEEN

(11) **EP 3 099 303 B1**
(45) Date de publication et mention de la délivrance du brevet: **08.04.2020**
(21) Numéro de dépôt: 15706688.7
(22) Date de dépôt: 28.01.2015
(51) Int. Cl.: A61K 31/517, A61K 31/536, A61P 17/02

(54) **TRAITEMENT DES DEGENERESCENCES ET DES LESIONS PHOTO-INDUITES DE LA RETINE**
BEHANDLUNG VON DEGENERATIONEN UND LICHTINDUZIERTEN SCHÄDEN DER NETZHAUT
TREATMENT OF DEGENERATIONS AND LIGHT-INDUCED DAMAGE TO THE RETINA

(30) Priorité: 29.01.2014 FR 1400223
(43) Date de publication de la demande: 07.12.2016
(73) Titulaire: Biocodex, 94250 Gentilly (FR)
(72) Inventeur: VERLEYE, Marc, F-60190 Remy (FR); GIRARD, Philippe, F-60280 Margny Les Compiegne (FR); LE GUERN, Marie-Emmanuelle, F-60200 Compiegne (FR)
(74) Mandataire: Vial, Lionel
(86) Numéro de dépôt international: PCT/EP2015/051669
(87) Numéro de publication internationale: WO 2015/113991

(56) Documents cités:
- WO-A1-02/078680
- WO-A1-2014/025792
- WO-A2-2007/109288
- US-A1- 2006 292 202
- MARC VERLEYE ET AL: "Effects of etifoxine on ligand binding to GABAA receptors in rodents", NEUROSCIENCE RESEARCH, ELSEVIER, SHANNON, IR, vol. 44, 1 janvier 2002 (2002-01-01), pages 167-172, XP007908404, ISSN: 0168-0102, DOI: 10.1016/S0168-0102(02)00121-9

## Description

### Domaine de l'invention

La présente invention concerne des composés et des compositions pharmaceutiques utiles dans la prévention ou le traitement des dégénérescences rétiniennes et/ou des lésions rétiniennes photo-induites, et en particulier dans la prévention ou le traitement de la dégénérescence maculaire liée à l'âge (DMLA).

### Arrière-plan technique

Les dégénérescences rétiniennes et les lésions photo-induites de la rétine regroupent des maladies telles que la photorétinite, la rétinite pigmentaire, la maculopathie liée à l'âge (MLA), et la dégénérescence maculaire liée à l'âge (DMLA).

Parmi ces maladies, la DMLA est d'importance particulière, puisqu'elle représente la première cause de malvoyance après 55 ans dans les pays industrialisés. La prévalence globale de la maladie est de 8% après 50 ans et augmente avec l'âge, passant d'environ 1 à 2% entre 50 et 65 ans, à 10% entre 65 et 75 ans, puis à 25% entre 75 et 85 ans.

L'étiologie de la DMLA est inconnue mais plusieurs facteurs de risque ont été mis en évidence ou sont suspectés, dont l'âge, le tabagisme, l'hypertension artérielle, la couleur claire de l'iris, les antécédents d'accidents coronariens, et l'exposition importante à la lumière. Par ailleurs, des facteurs génétiques jouent également un rôle dans la survenue de la maladie.

On distingue plusieurs stades dans la DMLA : des stades précoces, qui correspondent à la maculopathie liée à l'âge (MLA), ainsi que des stades plus tardifs, correspondant à la DMLA au sens strict, qui incluent les formes non-exsudatives, également dites sèches, ou atrophiques dans leur stade avancé, et les formes exsudatives, également dites humides ou néovasculaires.

En France, il n'y a, à l'heure actuelle, aucun traitement médicamenteux autorisé pour la forme atrophique de la DMLA. Quant à la forme exsudative de la DMLA, les anti-VEGF (*Vascular Endothelial Growth Factor,* facteur de croissance endothélial vasculaire) administrés par voie intravitréenne sont le seul traitement de première intention recommandé par la Haute Autorité de Santé (HAS). Il a ainsi été montré que le ranibizumab, un fragment Fab d'anticorps monoclonal anti-VEGF-A, en injections intravitréennes mensuelles de 0,3 mg et de 0,5 mg permettait un gain d'acuité visuelle de +8,1 et de +10,7 lettres respectivement chez des patients atteints de DMLA néovasculaire (exsudative), alors qu'une perte d'acuité visuelle de 9,8 lettres était observé chez les patients témoins traités par photothérapie dynamique (pour revue voir Fong & Lai (2013) Clinical Interventions in Aging 8:467-483).

En conséquence, il existe un besoin non pourvu pour la prise en charge pharmacologique de la forme non-exsudative, notamment atrophique, de la DMLA. De plus, s'agissant de la forme exsudative, il serait utile de disposer d'un traitement permettant de limiter, voire de se passer, des injections intravitréennes, particulièrement inconfortables pour le patient.

Dans ce cadre, au vu de l'action protectrice de la phényl-*N-tert*-butylnitrone vis-à-vis de la dégénérescence rétinienne induite par la lumière chez le rat après son administration intrapéritonéale (Ranchon et al. (2001) Investigative Ophthalmology & Visual Science 42:1375-1379), il a été proposé de l'utiliser pour le traitement de la DMLA, notamment dans sa forme sèche. Toutefois, ce composé n'a pas encore fait l'objet d'études cliniques pouvant conduire à son utilisation chez l'homme.

L'étifoxine, ou chlorhydrate de 6-chloro-2-éthylamino-4-méthyl-4-phényl-4H-[3,1]benzoxazine appartient à la famille des aminobenzoxazines. Elle favorise la transmission gabaergique en se liant sur un site proche du canal chlore couplé au récepteur GABA_{A} et est actuellement utilisée comme anxiolytique. Peu de manifestations indésirables consécutives à son utilisation sont répertoriées.

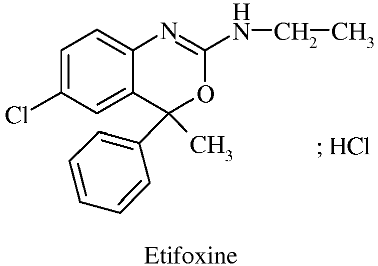

La synthèse de ce composé est notamment décrite dans le brevet français n° 1 571 287. Par ailleurs, plusieurs métabolites actifs de l'étifoxine ont été décrits, tels que la déséthyl-étifoxine ou 2-amino-6-chloro-4-méthyl-4-phényl-4H-[3,1]benzoxazine, la 6-chloro-4-(4-hydroxyphényl)-4-méthyl-3,4-dihydro-1H-quinazolin-2-one ou la 6-chloro-3-éthyl-7-hydroxy-4-méthyl-4-phényl-3,4-dihydro-1H-quinazolin-2-one.

### Résumé de l'invention

La présente invention découle de la mise en évidence inattendue que l'étifoxine permettait de protéger la rétine d'animaux soumis à un rayonnement lumineux induisant des lésions rétiniennes modélisant notamment la DMLA.

Ainsi, la présente invention concerne un composé de formule (I) suivante : 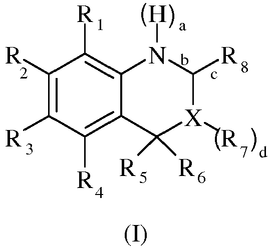 dans laquelle :
- a représente 0 ou 1 ;
- b représente une simple liaison ou une double liaison ;
- c représente une simple liaison ou une double liaison ;
- d représente 0 ou 1 ;
- X représente un atome d'oxygène ou d'azote, sous réserve que lorsque X représente un atome d'oxygène alors d vaut 0 et que lorsque X représente un atome d'azote alors d vaut 1 ;
- R₁, R₂, R₃, et R₄, identiques ou différents, représentent un atome d'hydrogène, un atome d'halogène, notamment choisi parmi F, Cl, Br, ou I, un groupe hydroxyle, ou un groupe alkoxyle de 1 ou 2 atomes de carbone ;
- R₅ et R₆, identiques ou différents, représentent un atome d'hydrogène, un groupe alkyle ou cycloalkyle de 1 à 6 atomes de carbone, ou un groupe aryle de 6 atomes de carbone dont le noyau aromatique est éventuellement substitué par un ou plusieurs atomes d'halogène ou un ou plusieurs groupes hydroxyle, alkoxyle de 1 ou 2 atomes de carbone, trifluorométhyle ou nitro ;
- R₇ représente un atome d'hydrogène, un groupe hydroxyle, ou un groupe alkyle ou hydroxyalkyle de 1 à 3 atomes de carbone ;
- R₈ représente un atome d'oxygène ou un groupe -NR₉R₁₀, R₉ et R₁₀, identiques ou différents, représentant un atome d'hydrogène, un groupe hydroxyle, ou un groupe alkyle ou hydroxyalkyle de 1 à 3 atomes de carbone, sous réserve que lorsque R₈ représente un atome d'oxygène alors a vaut 1, b représente une simple liaison et c représente une double liaison et que lorsque R₈ représente un groupe -NR₉R₁₀
alors a vaut 0, b représente une double liaison et c représente une simple liaison ; ou sel pharmaceutiquement acceptable de celui-ci,
pour son utilisation dans la prévention ou le traitement d'une lésion photo-induite et/ou d'une dégénérescence de la rétine chez un individu.

Dans un mode de réalisation particulier de l'invention, le composé ou le sel pharmaceutiquement acceptable de celui-ci pour son utilisation telle que définie ci-dessus est en combinaison avec au moins un autre composé utile pour la prévention ou le traitement d'une lésion photo-induite et/ou d'une dégénérescence de la rétine. Dans un autre mode de réalisation particulier de l'invention, le composé ou le sel pharmaceutiquement acceptable de celui-ci pour son utilisation telle que définie ci-dessus n'est pas en combinaison avec au moins un autre composé utile pour la prévention ou le traitement d'une lésion photo-induite et/ou d'une dégénérescence de la rétine

La présente invention concerne également une composition pharmaceutique, comprenant à titre de substance active au moins un composé de formule (I) tel que défini ci-dessus, ou un sel pharmaceutiquement acceptable de celui-ci, éventuellement en association avec un véhicule pharmaceutiquement acceptable, pour son utilisation dans la prévention ou le traitement d'une lésion photo-induite et/ou d'une dégénérescence de la rétine chez un individu.

Dans un mode de réalisation particulier de l'invention, la composition pharmaceutique pour son utilisation telle que définie ci-dessus comprend au moins un autre composé utile pour la prévention ou le traitement d'une lésion photo-induite et/ou d'une dégénérescence de la rétine. Dans un autre mode de réalisation particulier de l'invention, la composition pharmaceutique pour son utilisation telle que définie ci-dessus ne comprend pas d'autre composé utile pour la prévention ou le traitement d'une lésion photo-induite et/ou d'une dégénérescence de la rétine.

La présente invention concerne également une composition pharmaceutique, comprenant à titre de substances actives, au moins un composé de formule (I) tel que défini ci-dessus, ou un sel pharmaceutiquement acceptable de celui-ci, ainsi qu'au moins un autre composé utile pour la prévention ou le traitement d'une lésion photo-induite et/ou d'une dégénérescence de la rétine, éventuellement en association avec un véhicule pharmaceutiquement acceptable.

La présente invention concerne également des produits contenant :
- au moins un composé de formule (I) tel que défini ci-dessus, ou un sel pharmaceutiquement acceptable de celui-ci, et
- au moins un autre composé utile pour la prévention ou le traitement d'une lésion photo-induite et/ou d'une dégénérescence de la rétine,
comme produit de combinaison pour une utilisation, notamment simultanée, séparée ou étalée dans le temps, pour la prévention ou le traitement d'une lésion photo-induite et/ou d'une dégénérescence de la rétine chez un individu.

La présente invention concerne également une méthode pour prévenir ou traiter une lésion photo-induite ou une dégénérescence de la rétine chez un individu, comprenant l'administration à l'individu d'une quantité efficace d'un composé de formule (I) tel que défini ci-dessus, ou d'un sel pharmaceutiquement acceptable de celui-ci.

Dans un mode de réalisation particulier de l'invention, la méthode telle que définie ci-dessous comprend également l'administration d'au moins un autre composé utile pour la prévention ou le traitement d'une lésion photo-induite et/ou d'une dégénérescence de la rétine à l'individu.

La présente invention concerne également l'utilisation d'un composé de formule (I) tel que défini ci-dessus, ou d'un sel pharmaceutiquement acceptable de celui-ci, pour la préparation d'un médicament destiné à la prévention ou au traitement d'une lésion photo-induite et/ou d'une dégénérescence de la rétine chez un individu.

Dans un mode de réalisation particulier de l'invention, le médicament tel que défini ci-dessus comprend au moins un autre composé utile pour la prévention ou le traitement d'une lésion photo-induite et/ou d'une dégénérescence de la rétine.

### Description détaillée de l'invention

### Dégénérescence ou lésion photo-induite de la rétine

L'invention vise la prévention ou le traitement de toute dégénérescence de la rétine et/ou lésion photo-induite de la rétine, notamment en préservant ou en rétablissant le fonctionnement des photorécepteurs rétiniens.

De préférence, les dégénérescences et les lésions photo-induites de la rétine selon l'invention sont sélectionnées dans le groupe constitué de la photorétinite, de la rétinite pigmentaire, de la maculopathie liée à l'âge (MLA), ou de la dégénérescence maculaire liée à l'âge (DMLA). De manière particulièrement préférée, la lésion photo-induite et/ou la dégénérescence de la rétine selon l'invention, est la DMLA, plus particulièrement dans un stade modéré ou avancé, notamment sous sa forme non exsudative, autrement dit sèche, ou atrophique.

Ces maladies sont bien connues de l'homme du métier.

La photorétinite est une lésion de la rétine induite par la lumière, notamment dite « bleue », comprenant au moins une composante ayant une longueur d'onde comprise entre 400 nm et 550 nm.

La rétinite pigmentaire, également nommée rétinopathie pigmentaire ou *retinits pigmentosa,* est une dystrophie héréditaire de la rétine impliquant une dégénérescence de la rétine liée à la perte de photorécepteurs, cônes et/ou bâtonnets, et caractérisée par des dépôts pigmentaires visibles au fond d'œil. La rétinite pigmentaire au sens de l'invention peut être non syndromique ou syndromique, c'est à dire associée à des lésions d'autres organes. Ainsi, les rétinites pigmentaires au sens de l'invention couvrent également les syndromes tels que le syndrome d'Usher, le syndrome de Bardet-Biedl et la maladie de Refsum.

La maculopathie liée à l'âge (MLA) associe un ou plusieurs des éléments suivants entre eux :
- des drusen miliaires : petites tâches blanches asymptomatiques observées dans la région périmaculaire (diamètre inférieur à 63 µm) ;
- des drusen séreux : drusen de plus grande taille (supérieur à 125 µm), le plus souvent asymptomatiques mais pouvant s'accompagner d'une diminution des capacités visuelles à l'obscurité ;
- altérations pigmentaires à type d'hypopigmentation ou d'hyperpigmentation de l'épithélium pigmentaire. Elles traduisent la mort des cellules de l'épithélium pigmentaire.

La DMLA selon l'invention se présente sous deux formes, la DMLA exsudative, également nommée forme humide, et la DMLA non exsudative, également nommée forme sèche, ou atrophique dans un stade tardif.

La DMLA exsudative est caractérisée par la prolifération de néovaisseaux choroïdiens qui traversent la membrane de Bruch et se développent sous l'épithélium pigmentaire ou dans l'espace sous-rétinien. Il existe des formes particulières de néovaisseaux dans la DMLA exsudative, dont :
- les anastomoses chorio-rétiniennes, appelées aussi néovascularisation de type 3 ;
- les vasculopathies polypoïdales idiopathiques.

Les vasculopathies polypoïdales, qui sont liées à la formation d'un réseau vasculaire anormal d'origine choroïdienne qui se développe sous l'épithélium pigmentaire rétinien, se terminant par des dilatations polypoïdales ; ces lésions sont à l'origine de décollements séro-hémorragiques rétiniens et de l'épithélium pigmentaire localisés préférentiellement en inter-papillo-maculaire.

La DMLA atrophique ou atrophie géographique est la forme avancée de la DMLA non exsudative et se caractérise par une ou plusieurs plages d'atrophie de l'épithélium pigmentaire et/ou de la membrane choriocapillaire qui sont observées plus ou moins associées à des drusen (accumulation de dépôts lipidiques sous l'épithélium pigmentaire et dans la membrane de Bruch) et à des anomalies de l'épithélium pigmentaire. L'atrophie peut aussi être associée à une néovascularisation.

Les DMLA selon l'invention peuvent également être classées en quatre stades ou catégories définis par l'AREDS (*Age-Related Eye Disease Study*) :

| | |
|---|---|
| Catégorie 1 | Pas de dégénérescence maculaire liée à l'âge : aucun ou quelques petits drusen (diamètre inférieur à 63 µm) |
| Catégorie 2 : MLA | Un ou plusieurs des éléments suivants : |
| | - multiples petits drusen ; |
| | - quelques drusen de diamètre entre 63 et 125 µm ; |
| | - anomalie de l'épithélium pigmentaire. |
| Catégorie 3 : DMLA modérée | Un ou plusieurs des éléments suivants : |
| | - multiples drusen de diamètre entre 63 et 125 µm et au moins un drusen de diamètre supérieur à 125 µm ; |
| | - atrophie géographique excluant la fovéa |
| Catégorie 4 : DMLA avancée | Atrophie géographique touchant la fovéa et/ou présence d'une dégénérescence maculaire liée à l'âge exsudative. |

Ainsi, de manière tout particulièrement préférée, la lésion photo-induite et/ou la dégénérescence de la rétine selon l'invention est une DMLA non exsudative de catégorie 3 ou 4 selon la classification AREDS, c'est-à-dire une DMLA non exsudative modérée ou avancée.

### Individu

L'individu au sens de l'invention est de préférence un être humain, plus préférentiellement âgés de 50 ans ou plus.

Dans un mode de réalisation préféré de l'invention, l'individu selon l'invention suit une photothérapie dynamique. La photothérapie dynamique est bien connue de l'homme du métier ; elle associe une radiation laser à faible intensité après injection d'un produit sensibilisant, notamment la vertéporfine, qui se fixe électivement sur la paroi des néovaisseaux.

Lorsque l'invention vise le traitement d'une lésion photo-induite ou d'une dégénérescence de la rétine on préfère que l'individu soit atteint d'une MLA, d'une DMLA modérée ou de catégorie 3, ou d'une DMLA avancée ou de catégorie 4, plus particulièrement d'une DMLA avancée ou de catégorie 4, notamment sous sa forme non exsudative.

Par ailleurs, lorsque l'invention vise la prévention d'une lésion photo-induite ou d'une dégénérescence de la rétine on préfère que l'individu présente un score du risque de progression d'une forme précoce de DMLA vers une forme tardive à cinq ans d'au moins 1, plus préférablement d'au moins 2, encore plus préférablement d'au moins 3, et de manière tout particulièrement préférée d'au moins 4.

Comme cela est bien connu de l'homme du métier et comme cela est notamment décrit dans la publication intitulée «Dégénérescence maculaire liée à l'âge : prise en charge diagnostique et thérapeutique » de juin 2012 de la Haute Autorité de Santé (HAS) (France), l'évaluation du risque de progression d'une forme précoce de DMLA vers une forme tardive à cinq ans est proposée sous forme d'un score de 0 à 4 calculé en fonction des anomalies présentes sur un ou les deux yeux :

| |
|---|
| Un score de 1 est attribué par œil pour la présence de larges drusen ou d'anomalies pigmentaires. |
| Le score est de 2 si les deux éléments sont présents (présence de larges drusen ou d'anomalies pigmentaires). |
| Un score de 2 est attribué à un œil présentant une DMLA avancée (atrophie géographique touchant la fovéa et/ou présence d'une DMLA exsudative). |
| Le score de chacun des deux yeux est additionné et correspond à un niveau de risque. |
| Le score obtenu est ensuite corrélé à un pourcentage de risque à cinq ans de développer une DMLA avancée : |
| - un score de 0 correspond à un risque de 0,5 % ; |
| - un score de 1 correspond à un risque de 3 % ; |
| - un score de 2 correspond à un risque de 12 % ; |
| - un score de 3 correspond à un risque de 25 % ; |
| - un score de 4 correspond à un risque de 50 %. |

### Composé de formule (I)

La synthèse des composés de formule (I) définie ci-dessus peut aisément être mise en œuvre à partir des enseignements du brevet français n° 1 571 287.

Les sels pharmaceutiquement acceptables selon l'invention apparaîtront de manière évidente pour l'homme du métier. En particulier les sels de chlorhydrate des composés de formule (I) selon l'invention sont préférés.

Comme on l'entend ici, la formule (I) définie ci-dessus englobe notamment les formules des composés de formule (I) optiquement actifs, tels que les énantiomères représentés par les formules suivantes (lorsque R₅ et R₆ sont différents) : 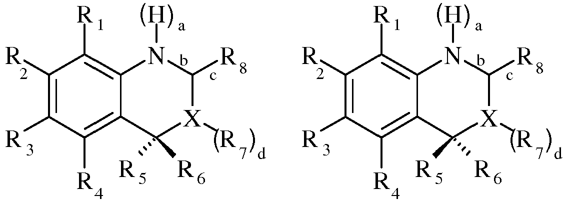 ou leurs mélanges, en particulier leur mélange racémique.

Dans un mode de réalisation préférentiel de l'invention, dans la formule (I) définie ci-dessus, R₅ et R₆, identiques ou différents, représentent un groupe alkyle ou cycloalkyle de 1 à 6 atomes de carbone, ou un groupe aryle de 6 atomes de carbone dont le noyau aromatique est éventuellement substitué par un ou plusieurs atomes d'halogène ou un ou plusieurs groupes hydroxyle, alkoxyle de 1 ou 2 atomes de carbone, trifluorométhyle ou nitro.

Dans un mode de réalisation préférentiel de l'invention, la formule (I) définie ci-dessus est représentée par la formule (VIII) suivante : 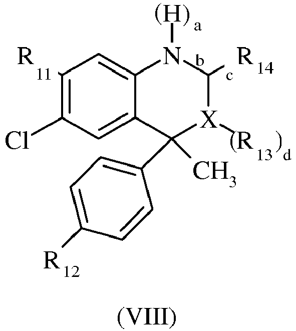 dans laquelle :
- a représente 0 ou 1 ;
- b représente une simple liaison ou une double liaison ;
- c représente une simple liaison ou une double liaison ;
- d représente 0 ou 1 ;
- X représente un atome d'oxygène ou d'azote, sous réserve que lorsque X représente un atome d'oxygène alors d vaut 0 et que lorsque X représente un atome d'azote alors d vaut 1 ;
- R₁₁ et R₁₂ identiques ou différents représentent -H ou -OH ;
- R₁₃ représente -H ou un groupe -CH₂-CH₃ ;
- R₁₄ représente un atome d'oxygène ou un groupe -NH₂ ou -NH-CH₂-CH₃, sous réserve que lorsque R₁₄ représente un atome d'oxygène alors a vaut 1, b représente une simple liaison et c représente une double liaison et que lorsque R₁₄ représente un groupe -NH₂ ou -NH-CH₂-CH₃ alors a vaut 0, b représente une double liaison et c représente une simple liaison.

Comme on l'entend ici, la formule (VIII) définie ci-dessus englobe notamment les formules des composés de formule (VIII) optiquement actifs, tels que les énantiomères représentés par les formules suivantes : 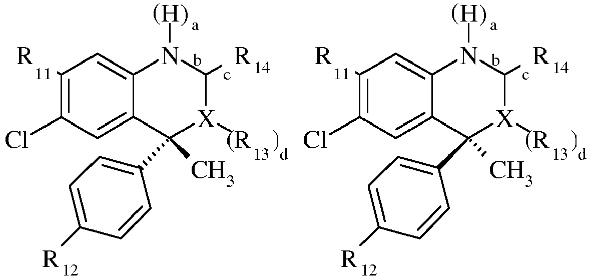 ou leurs mélanges, en particulier leur mélange racémique.

Dans un autre mode de réalisation préférentiel de l'invention, la formule (I) définie ci-dessus est représentée par la formule (II) suivante : 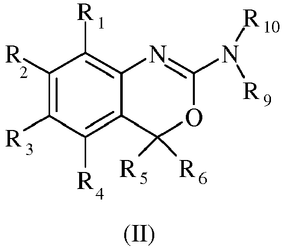 dans laquelle R₁, R₂, R₃, R₄, R₅, R₆, R₉ et R₁₀ sont tels que définis ci-dessus.

Comme on l'entend ici, la formule (II) définie ci-dessus englobe notamment les formules des composés de formule (II) optiquement actifs, tels que les énantiomères représentés par les formules suivantes (lorsque R₅ et R₆ sont différents) : 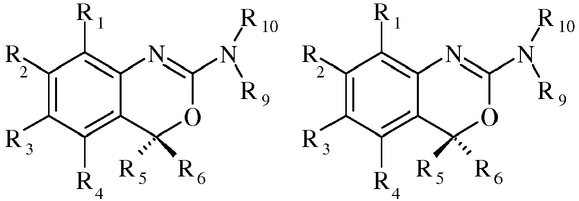 ou leurs mélanges, en particulier leur mélange racémique.

Dans un autre mode de réalisation préférentiel de l'invention, la formule (I) définie ci-dessus est représentée par la formule (IIIa), (IIIb) ou (IV) suivante :

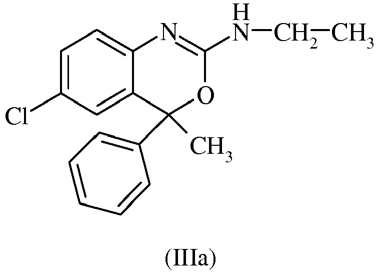

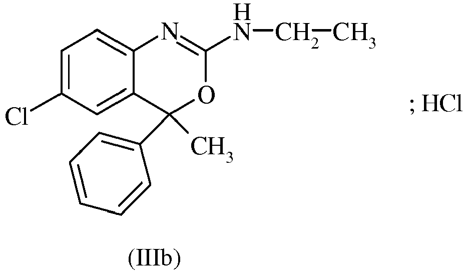

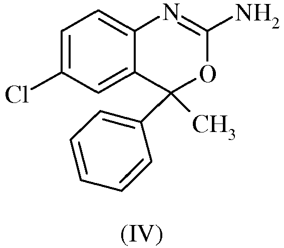

Le composé de formule (IIIa) est l'étifoxine base ou 6-chloro-2-éthylamino-4-méthyl-4-phényl-4H-[3,1]benzoxazine. Le composé de formule (IIIb) est l'étifoxine, ou chlorhydrate de 6-chloro-2-éthylamino-4-méthyl-4-phényl-4H-[3,1]benzoxazine.

Le composé de formule (IV), la déséthyl-étifoxine ou 2-amino-6-chloro-4-méthyl-4-phényl-4H-[3,1]benzoxazine, est un métabolite de l'étifoxine.

Comme on l'entend ici, la formule (IIIa) ou (IIIb) définie ci-dessus englobe notamment les formules des composés de formule (IIIa) ou (IIIb) optiquement actifs, tels que les énantiomères représentés par les formules suivantes : 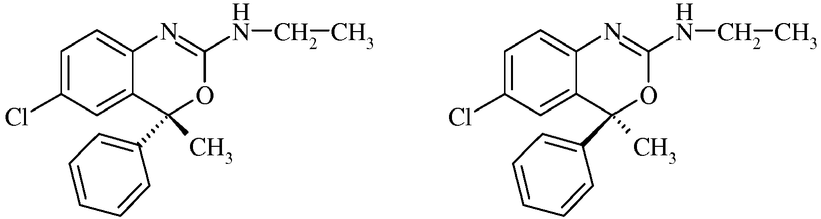 ou leurs mélanges, en particulier leur mélange racémique, notamment sous forme chlorhydrate, ainsi que les formules des composés de formule (IV) optiquement actifs, tels que les énantiomères représentés par les formules suivantes : 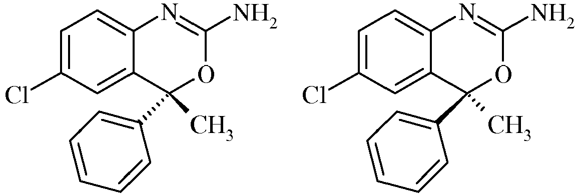 ou leurs mélanges, en particulier leur mélange racémique.

Dans un autre mode de réalisation préférentiel de l'invention, la formule (I) est représentée par la formule (V) suivante : 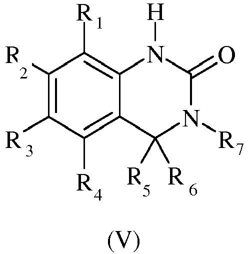 dans laquelle R₁, R₂, R₃, R₅, R₆, et R₇ sont tels que définis ci-dessus.

Comme on l'entend ici, la formule (V) définie ci-dessus englobe notamment les formules des composés de formule (V) optiquement actifs, tels que les énantiomères représentés par les formules suivantes (lorsque R₅ et R₆ sont différents) : 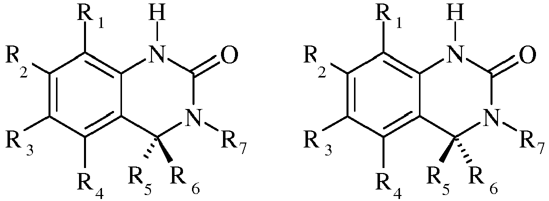 ou leurs mélanges, en particulier leur mélange racémique.

Dans un autre mode de réalisation préférentiel de l'invention, la formule (I) est représentée par la formule (VI) ou (VII) suivante :

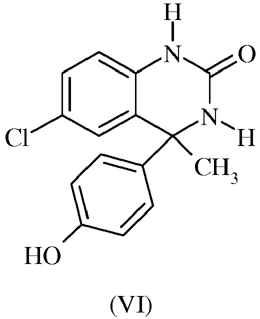

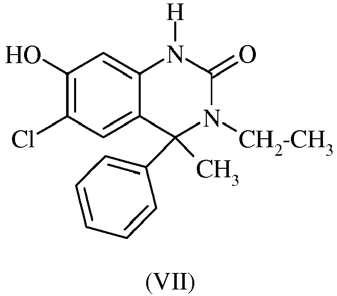

Les composés de formule (VI) (6-chloro-4-(4-hydroxy-phényl)-4-méthyl-3,4-dihydro-1H-quinazolin-2-one) et (VII) (6-chloro-3-éthyl-7-hydroxy-4-méthyl-4-phényl-3,4-dihydro-1H-quinazolin-2-one) sont des métabolites de l'étifoxine.

Comme on l'entend ici, la formule (VI) définie ci-dessus englobe notamment les formules des composés de formule (VI) optiquement actifs, tels que les énantiomères représentés par les formules suivantes : 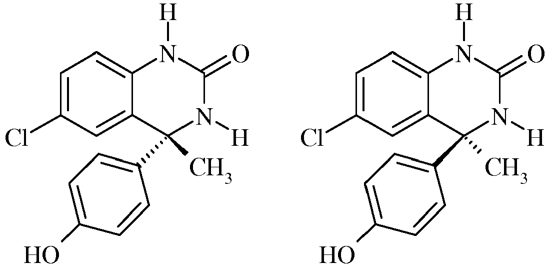 ou leurs mélanges, en particulier leur mélange racémique, ainsi que les formules des composés de formule (VII) optiquement actifs, tels que les énantiomères suivants : 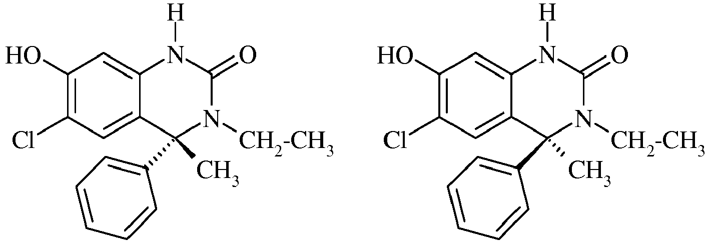 ou leurs mélanges, en particulier leur mélange racémique.

### Composé additionnel

L'autre composé utile pour la prévention ou le traitement d'une lésion photo-induite et/ou d'une dégénérescence de la rétine selon l'invention peut être de tout type, cependant on préfère qu'il soit sélectionné dans le groupe constitué d'un anti-VEGF, d'un corticoïde et d'un antioxydant.

Les anti-VEGF sont bien connus de l'homme du métier, il s'agit généralement de ligands de l'isoforme A du VEGF, qui regroupent, notamment, le ranibizumab, le pegaptanib, le bevacizumab et l'aflibercept.

Les corticoïdes selon l'invention sont de préférence la triamcinolone et l'acétate d'anécortave.

Les antioxydants selon l'invention sont de préférence des anthocyanosides, le β-carotène et l'α-tocophérol.

### Administration

De préférence, le composé de formule (I) telle que définie ci-dessus, ou son sel pharmaceutiquement acceptable, est administré ou administrable à une dose unitaire, ou est conditionné en une dose unitaire, d'environ 50 mg à environ 1500 mg, notamment d'environ 150 à environ 200 mg. De préférence également, le composé de formule (I) telle que définie ci-dessus, ou son sel pharmaceutiquement acceptable est administré ou administrable avec un régime de dosage de 50 mg/j à environ 1500 mg/j, notamment d'environ 150 mg/j à environ 200 mg/j.

De préférence, le composé de formule (I) telle que définie ci-dessus, ou son sel pharmaceutiquement est administré ou administrable sous une forme convenant pour une administration par voie orale ou injectable. Plus préférablement, le composé de formule (I) telle que définie ci-dessus, ou son sel pharmaceutiquement acceptable, est administré ou administrable sous la forme d'une poudre, de cachets, de gélules ou de sachets. De préférence, le composé de formule (I) telle que définie ci-dessus, ou son sel pharmaceutiquement acceptable, n'est pas inclus dans un réservoir d'une matrice de type hydrogel en vue de son administration à la surface d'un œil d'un individu.

Comme on l'entend ici l'expression « en combinaison » ou « produit de combinaison » signifie que le composé de formule (I) telle que définie ci-dessus, ou un sel pharmaceutiquement acceptable de celui-ci, et l'autre composé utile pour la prévention ou le traitement d'une lésion photo-induite et/ou d'une dégénérescence de la rétine tel que défini ci-dessus peuvent être associés au sein d'une même composition pharmaceutique ou d'un même médicament, et donc être administrés ensemble, ou bien être administrés de manière séparée, c'est-à-dire selon des voies d'administration distinctes et/ou des régimes d'administration distincts, sous réserve que lorsqu'ils sont administrés de manière séparée les périodes du composé de formule (I) telle que définie ci-dessus et de l'autre composé utile pour la prévention ou le traitement d'une lésion photo-induite et/ou d'une dégénérescence de la rétine tel que défini ci-dessus se recouvrent en totalité ou en partie.

Ainsi, lorsque les composés sont administrés de manière séparée, le composé de formule (I) telle que définie ci-dessus, ou son sel pharmaceutiquement acceptable, sera de préférence administré dans les 24 heures, plus préférablement dans les 2 heures, et encore plus préférablement dans l'heure, suivant l'administration de l'autre composé utile pour la prévention ou le traitement d'une lésion photo-induite et/ou d'une dégénérescence de la rétine tel que défini ci-dessus, et son administration sera éventuellement poursuivie les jours suivants. Réciproquement, l'autre composé utile pour la prévention ou le traitement d'une lésion photo-induite et/ou d'une dégénérescence de la rétine tel que défini ci-dessus sera de préférence administré dans les 24 heures, plus préférablement dans les 2 heures, et encore plus préférablement dans l'heure, suivant l'administration du composé de formule (I) telle que définie ci-dessus, ou de son sel pharmaceutiquement acceptable, et son administration sera éventuellement poursuivie les jours suivants. Dans un autre mode de réalisation préférée de l'invention, lorsque le composé de formule (I) telle que définie ci-dessus et l'autre composé utile pour la prévention ou le traitement d'une lésion photo-induite et/ou d'une dégénérescence de la rétine tel que défini ci-dessus sont administrés de manière séparée, ils sont administrés essentiellement simultanément.

L'invention sera davantage explicitée à l'aide de l'Exemple et des Figures non limitatifs qui suivent.

### Description des figures

Figure 1

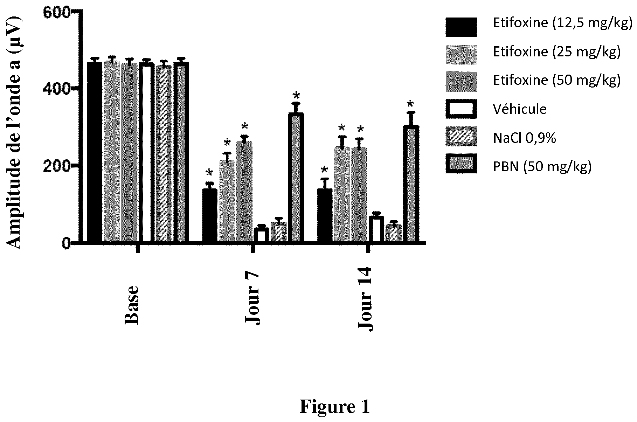

   La figure 1 représente l'amplitude moyenne de l'onde a (axe des ordonnées, en µV) avant l'induction de la dégénérescence rétinienne (base), 7 jours après l'induction et 14 jours après l'induction pour les groupes de rats traités à l'étifoxine administrée à 12,5 mg/kg (colonnes noires), à 25 mg/kg (colonnes en gris claire), à 50 mg/kg (colonnes en gris foncé), ou avec le véhicule seul (colonnes blanches), avec une solution de NaCl à 0,9% (colonnes hachurées) ou avec la PBN (colonnes grises entourées de noir). Le symbole étoile (*) représente p<0,05 par rapport au groupe véhicule ou NaCl 0,9%.
Figure 2

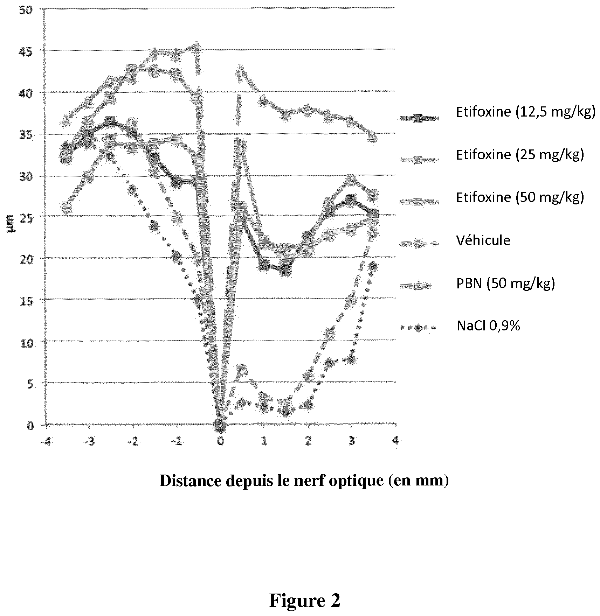

   La figure 2 représente l'épaisseur moyenne de la couche nucléaire externe 14 jours après l'induction de la dégénérescence rétinienne (axe des ordonnées, en µm) en fonction de la distance par rapport au nerf optique (axe des abscisses, en mm). La courbe en gris foncé/points carrés/trait continu représente le groupe de rats traités par de l'étifoxine à 12,5 mg/kg, la courbe en gris clair/points carrés/trait continu représente le groupe de rats traités par de l'étifoxine à 25 mg/kg, la courbe en gris moyen/points carrés/trait continu représente le groupe de rats traités par de l'étifoxine à 50 mg/kg, la courbe en gris clair/points ronds/trait discontinu représente le groupe de rats traités par le véhicule, la courbe en gris clair/points triangulaires/trait discontinu représente le groupe de rats traités par la PBN à 50 mg/kg et la courbe en gris clair/points en losange/trait en pointillé représente le groupe de rats traités par une solution de NaCl à 0,9%.

### Exemple

Les inventeurs ont étudié l'effet protecteur de l'étifoxine vis-à-vis des dégénérescences ou des lésions rétiniennes induites par la lumière bleue chez le rat. Ce modèle animal est notamment représentatif, en plus des lésions rétiniennes induites par la lumière, de la dégénérescence maculaire liée à l'âge (DMLA) et de la rétinite pigmentaire (voir par exemple Feng et al. (2012) International Journal of Ophtalmology 5:151-157).

### I. Matériels et méthodes

### 1.1. Produits

Le Tween 80 utilisé comme véhicule de l'étifoxine, a été obtenu auprès de Sigma et dilué à 1% (v/v) dans une solution stérile de NaCl à 0,9% (Fournisseur : Aguettant). L'étifoxine (chlorhydrate de 6-chloro-2-éthylamino-4-méthyl-4-phényl-4H-[3,1]benzoxazine) (également nommée EFX dans la suite) a été obtenue sous forme de poudre auprès de Biocodex. Moins de 6 heures avant administration l'étifoxine est mise en suspension dans la solution véhicule à 1% de Tween 80 aux concentrations de 2,5 mg/mL, 5 mg/ml et 10 mg/mL et conservée entre 2°C et 8°C.

La phényl-*N-tert*-butylnitrone (PBN) (produit de référence) a été obtenue sous forme de poudre auprès de Sigma. Moins de 12 heures avant administration, la PBN est mise en solution dans une solution saline stérile de NaCl à 0,9% à la concentration de 20 mg/mL et protégée de la lumière pendant sa conservation à une température comprise entre 2°C et 8°C.

### 1.2. Animaux

Les animaux utilisés ont été traités conformément à la Directive 2010/63/EU et aux recommandations de l'Association pour la Recherche en Vision et en Ophtalmologie (ARVO).

72 rats mâles albinos Sprague-Dawley obtenus auprès de Janvier ont été utilisés pour l'étude. A réception les rats étaient âgés d'environ 7 semaines et pesaient 180-200 g.

Les rats ont été maintenus en observation pendant 3 semaines après leur arrivée afin de les acclimater. Pendant ce temps les animaux ont été observés quotidiennement pour vérifier l'absence de maladie. Les animaux inclus dans l'étude étaient dépourvus d'anomalies oculaires.

En dehors de l'induction (exposition à la lumière bleue), les animaux ont été maintenus à 2 ou 3 dans des cages standards dans des conditions environnementales identiques : température à 22 ± 2°C ; humidité à 55 ± 10% ; cycle jour (10-200 lx) - nuit de 12h/12h. Les animaux ont eu un accès libre à l'eau (robinet) et à la nourriture (LASvendi GmbH).

### 1.3. Procédure expérimentale

Les 72 rats ont été divisés en 6 groupes de 12 animaux qui ont été soumis aux traitements résumés dans le tableau ci-dessous :

| **Groupe** | **Traitement** | **Dose** | **Voie d'administration** | **Régime de dosage** |
|---|---|---|---|---|
| 1 | Etifoxine (EFX) | 12,5 mg/kg | Intrapéritonéale 0,5 mL/100 g | 1 fois par jour de j-7 à j0 30 min. avant l'induction à j0 |
| 2 | | 25 mg/kg | | |
| 3 | | 50 mg/kg | | |
| 4 | Véhicule | - | | |
| 5 | NaCl 0,9% | - | Intrapéritonéale 0,25 mL/100 g | A j0, 0,5h avant l'induction, à 2h et 4h pendant l'induction et juste après l'induction |
| 6 | PBN | 50 mg/kg | | |

L'induction a été réalisée par une exposition pendant 6 heures à une lumière bleue fluorescente de longueur d'onde 400 nm - 540 nm en cages individuelles. Les rats ont été maintenus à l'obscurité 36 heures préalablement et 24 postérieurement à l'induction avant de retourner aux conditions d'éclairage normales.

Un électrorétinogramme (ERG) scotopique a été réalisé à l'aide du système électrophysiologique RETI-animal® (Roland Consult) avant la première administration (base) puis aux jours 7 et 14 sur les deux yeux d'animaux habitués à l'obscurité. Les animaux ont été anesthésiés par une injection intramusculaire d'un mélange kétamine/xylazine. 15 minutes avant la mesure, 1 goutte de Mydriaticum® (tropicamide à 0,5%) a été instillée pour induire une dilatation de la pupille. Les amplitudes de l'onde a et de l'onde b ont été mesurées pour chaque œil et exprimées en pourcentage des amplitudes de base.

Les paramètres de l'ERG sont les suivants :
- Gansfeld Q450C
- Couleur : blanc maximum
- Intensité maximale : 2,6 cd.s/m² (0 dB) ; durée : 0,24 ms ; nombre de flash : 1
- Filtre : 50 Hz
- Seuil d'impédance : 90 kΩ

Après l'ERG (jour 14), les animaux ont été euthanasiés à l'aide d'une surdose de pentobarbital, conformément aux recommandations européennes, les deux yeux ont été énucléés, fixés à l'aide de liquide de Bouin Hollande, puis inclus dans de la paraffine. Des sections de 5 à 7 µm d'épaisseur ont été réalisées le long du méridien vertical puis teintées à l'aide de trichrome de Masson. Le méridien vertical inclut le nerf optique. L'épaisseur de la couche nucléaire externe (*outer nuclear layer,* ONL) a été déterminée tous les 500 µm, pour sept points, depuis le nerf optique, en direction de la rétine périphérique, à la fois dans la rétine supérieure et dans la rétine inférieure, à l'aide d'un microscope standard (Leica). L'aire sous la courbe (*area under the curve,* AUC) a alors été calculée pour chaque rétine.

Les analyses statistiques ont été réalisées à l'aide du logiciel GraphPad Prism 6.0.

Un test ANOVA à deux facteurs a été appliqué aux amplitudes moyennes de l'onde a des deux yeux pour chaque point de mesure. L'effet des produits a été déterminé à l'aide du test de Tukey pour les comparaisons multiples ; chaque groupe a été comparé aux autres groupes. Les différences ont été considérées significatives pour les valeurs de p inférieures à 0,05.

Un test ANOVA à un facteur a été appliqué aux AUC moyennes de l'épaisseur de la couche nucléaire externe. L'effet des produits a été déterminé à l'aide du test de Tukey pour les comparaisons multiples ; chaque groupe traité a été comparé au groupe véhicule. Les différences ont été considérées significatives pour les valeurs de p inférieures à 0,05.

### II. Résultats

### II.1. Comportement des animaux et poids corporel

Aucun signe clinique inattendu n'a été enregistré après l'administration d'étifoxine.

Aucune différence notable de prise de poids n'a été mise en évidence entre les différents groupes de rats.

### II.2. Electrorétinogrammes

Les électrorétinogrammes (ERG) permettent de mesurer l'amplitude de l'onde a, qui résulte de l'hyperpolarisation photo-activée des photorécepteurs. Ainsi, une réduction de l'amplitude de l'onde a est un indicateur d'une lésion ou d'une dégénérescence rétinienne liée à l'exposition à la lumière bleue.

Les amplitudes de l'onde a des deux yeux ont été moyennées pour chaque rat et les valeurs moyennes sont présentées dans la **Figure 1** et le **Tableau 1** ci-dessous :

**Tableau 1 : amplitude et amplitude relative de l'onde a**

| **Traitement** | **Base** | **Jour 7** | | **Jour 14** | |
|---|---|---|---|---|---|
| | Amplitude de l'onde a (µV, moyenne ± écart type) | Amplitude de l'onde a (µV, moyenne ± écart type) | Amplitude relative moyenne de l'onde a (%) | Amplitude de l'onde a (µV, moyenne ± écart type) | Amplitude relative moyenne de l'onde a (%) |
| EFX 12,5mg/kg | 465±68 | 136±89 | 32±23 | 134±135 | 33±36 |
| EFX 25 mg/kg | 468±70 | 210±116 | 45±24 | 245±143 | 53±31 |
| EFX 50 mg/kg | 461±82 | 260±87 | 59±21 | 244±138 | 52±26 |
| Véhicule | 463±64 | 35±53 | 8±13 | 66±58 | 15±14 |
| NaCl 0,9% | 455±78 | 50±68 | 10±12 | 44±55 | 9±11 |
| PBN 50 mg/kg | 465±67 | 334±137 | 71±29 | 301±187 | 62±38 |

| | | | | | |
|---|---|---|---|---|---|
| N.B. : l'amplitude relative est un pourcentage de l'amplitude basale | | | | | |

L'amplitude de l'onde a est réduite de manière significative (p<0,0001) à 8 à 15% des niveaux basaux 7 et 14 jours après l'exposition pour les groupes véhicule et NaCl 0,9% (témoins négatifs), ce qui valide l'induction. Par ailleurs, les animaux traités par le produit de référence PBN (témoins positif) présentent des amplitudes de l'onde a significativement (p<0,0001) supérieures à celles du groupe NaCl 0,9%, aux jours 7 et 14, ce qui valide la procédure de traitement.

S'agissant des traitements à l'étifoxine, on observe une protection dose-dépendante significative dès 7 jours après l'induction (p<0,0001 pour toutes les doses par rapport au groupe véhicule), ainsi que 14 jours après l'induction (p=0,016 pour 12,5 mg/kg et p<0,0001 pour 25 et 50 mg/kg par rapport au groupe véhicule).

### II.3. Mesure de l'épaisseur de la couche nucléaire externe

Afin de déterminer si le traitement permet de préserver la structure des photorécepteurs, l'épaisseur de la couche nucléaire externe a été évaluée 14 jours après l'exposition à la lumière bleue. L'aire sous la courbe (AUC) de l'épaisseur de la couche nucléaire externe a été calculée pour chaque œil et les deux AUC ont été moyennées pour chaque rat.

La **Figure 2** illustre l'épaisseur moyenne de la couche nucléaire externe le long de la rétine. Par ailleurs, l'intégration de l'aire sous la courbe représentative de l'épaisseur de la couche nucléaire externe a été calculée pour comparer les différents groupes traités. La moyenne des deux yeux de l'AUC ainsi calculée est présentée dans le **Tableau 2** ci-dessous :

**Tableau 2 : Aire sous la courbe moyenne de l'épaisseur de la couche nucléaire externe**

| **Traitement** | **Epaisseur de la couche nucléaire externe (AUC, µm x mm, moyenne des 2 yeux ± écart type)** |
|---|---|
| EFX 12,5mg/kg | 170±72 (p=0,04 par rapport au groupe véhicule) |
| EFX 25 mg/kg | 200±73 (p=0,0002 par rapport au groupe véhicule) |
| EFX 50 mg/kg | 172±61 (p=0,035 par rapport au groupe véhicule) |
| Véhicule | 116±51 |
| NaCl 0,9% | 91±59 |
| PBN 50 mg/kg | 247±44 (p<0,0001 par rapport au groupe NaCl 0,9%) |
| Rats non induits (n=5) | 274±18,83 |

Au contraire de la solution de NaCl à 0,9% (témoins négatif), la PBN (témoins positif) protège la couche nucléaire externe des lésions ou des dégénérescences induites par la lumière bleue, comme le montre la comparaison avec les rats non induits, ce qui valide la procédure expérimentale. On observe par ailleurs que l'hémisphère supérieur (de 0 à +4 mm) est plus endommagé que l'hémisphère inférieur (de -4 à 0 mm). En outre, aucune différence n'est observable entre les groupes véhicule et NaCl à 0,9%.

S'agissant de l'étifoxine, quelles que soient les doses, celle-ci protège de manière significative, par rapport au véhicule, la couche nucléaire externe des lésions ou dégénérescences induites par la lumière bleue.

En conclusion, l'étifoxine protège les photorécepteurs rétiniens des lésions ou dégénérescences induites par la lumière bleue, ce qui est indicatif d'un effet protecteur de ce composé vis-à-vis des dégénérescences rétiniennes et/ou des lésions rétiniennes photo-induites, telles que la dégénérescence maculaire liée à l'âge (DMLA).

## Revendications

1. Composé de formule (I) suivante : 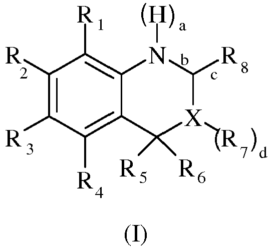 dans laquelle :
- a représente 0 ou 1 ;
- b représente une simple liaison ou une double liaison ;
- c représente une simple liaison ou une double liaison ;
- d représente 0 ou 1 ;
- X représente un atome d'oxygène ou d'azote, sous réserve que lorsque X représente un atome d'oxygène alors d vaut 0 et que lorsque X représente un atome d'azote alors d vaut 1 ;
- R₁, R₂, R₃, et R₄, identiques ou différents, représentent un atome d'hydrogène, un atome d'halogène, notamment choisi parmi F, Cl, Br, ou I, un groupe hydroxyle, ou un groupe alkoxyle de 1 ou 2 atomes de carbone ;
- R₅ et R₆, identiques ou différents, représentent un atome d'hydrogène, un groupe alkyle ou cycloalkyle de 1 à 6 atomes de carbone, ou un groupe aryle de 6 atomes de carbone dont le noyau aromatique est éventuellement substitué par un ou plusieurs atomes d'halogène ou un ou plusieurs groupes hydroxyle, alkoxyle de 1 ou 2 atomes de carbone, trifluorométhyle ou nitro ;
- R₇ représente un atome d'hydrogène, un groupe hydroxyle, ou un groupe alkyle ou hydroxyalkyle de 1 à 3 atomes de carbone ;
- R₈ représente un atome d'oxygène ou un groupe -NR₉R₁₀, R₉ et R₁₀, identiques ou différents, représentant un atome d'hydrogène, un groupe hydroxyle, ou un groupe alkyle ou hydroxyalkyle de 1 à 3 atomes de carbone, sous réserve que lorsque R₈ représente un atome d'oxygène alors a vaut 1, b représente une simple liaison et c représente une double liaison et que lorsque R₈ représente un groupe -NR₉R₁₀ alors a vaut 0, b représente une double liaison et c représente une simple liaison ;
ou sel pharmaceutiquement acceptable de celui-ci,
pour son utilisation dans la prévention ou le traitement d'une lésion photo-induite et/ou d'une dégénérescence de la rétine chez un individu.

2. Composé ou sel pharmaceutiquement acceptable de celui-ci pour son utilisation selon la revendication 1, dans lequel la formule (I) est représentée par la formule (II) suivante : 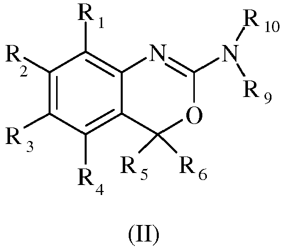 dans laquelle R₁, R₂, R₃, R₄, R₅, R₆, R₉ et R₁₀ sont tels que définis ci-dessus.

3. Composé ou sel pharmaceutiquement acceptable de celui-ci pour son utilisation selon la revendication 1 ou 2, de formule (IIIa), (IIIb) ou (IV) suivante :

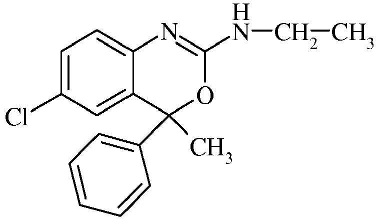

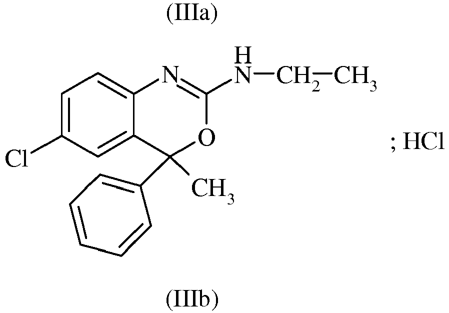

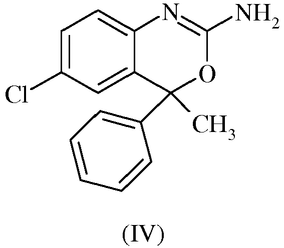

4. Composé ou sel pharmaceutiquement acceptable de celui-ci pour son utilisation selon la revendication 1, dans lequel la formule (I) est représentée par la formule (V) suivante : 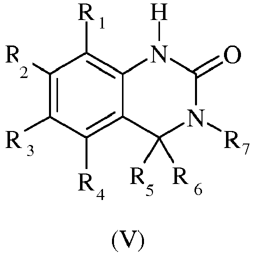 dans laquelle R₁, R₂, R₃, R₅, R₆, et R₇ sont tels que définis dans la revendication 1.

5. Composé ou sel pharmaceutiquement acceptable de celui-ci pour son utilisation selon la revendication 1 ou 4, de formule (VI) ou (VII) suivante :

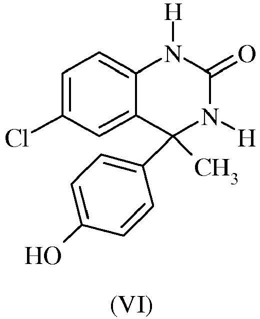

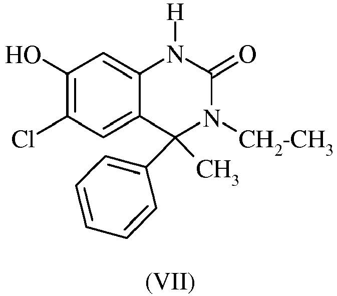

6. Composé ou sel pharmaceutiquement acceptable de celui-ci pour son utilisation selon l'une des revendications 1 à 5, conditionné en une dose unitaire de 50 mg à 1500 mg.

7. Composé ou sel pharmaceutiquement acceptable de celui-ci pour son utilisation selon l'une des revendications 1 à 6, sous une forme convenant pour une administration par voie orale.

8. Composé ou sel pharmaceutiquement acceptable de celui-ci pour son utilisation selon l'une des revendications 1 à 7, sous forme d'une poudre, de cachets, de gélules ou de sachets.

9. Composé ou sel pharmaceutiquement acceptable de celui-ci pour son utilisation selon l'une des revendications 1 à 8, dans la prévention ou le traitement de la photorétinite, de la rétinite pigmentaire, de la maculopathie liée à l'âge (MLA), ou de la dégénérescence maculaire liée à l'âge (DMLA).

10. Composé ou sel pharmaceutiquement acceptable de celui-ci pour son utilisation selon l'une des revendications 1 à 9, en combinaison avec au moins un autre composé utile pour la prévention ou le traitement d'une lésion photo-induite et/ou d'une dégénérescence de la rétine sélectionné dans le groupe constitué d'un anti-VEGF, d'un corticoïde et d'un antioxydant.

11. Composé ou sel pharmaceutiquement acceptable de celui-ci pour son utilisation selon l'une des revendications 1 à 10, dans laquelle l'individu suit une photothérapie dynamique.

12. Composition pharmaceutique, comprenant à titre de substance active, au moins un composé de formule (I) tel que défini dans l'une des revendications 1 à 5, ou un sel pharmaceutiquement acceptable de celui-ci,
éventuellement en association avec un véhicule pharmaceutiquement acceptable, et comprenant optionnellement au moins un autre composé utile pour la prévention ou le traitement d'une lésion photo-induite et/ou d'une dégénérescence de la rétine, notamment sélectionné dans le groupe constitué d'un anti-VEGF, d'un corticoïde et d'un antioxydant,
pour son utilisation dans la prévention ou le traitement d'une lésion photo-induite et/ou d'une dégénérescence de la rétine, et plus particulièrement dans la prévention ou le traitement de la photorétinite, de la rétinite pigmentaire, de la maculopathie liée à l'âge (MLA), ou de la dégénérescence maculaire liée à l'âge (DMLA), chez un individu, notamment un individu qui suit une photothérapie dynamique.

13. Composition pharmaceutique, comprenant à titre de substance active, au moins un composé de formule (I) tel que défini dans l'une des revendications 1 à 5, ou un sel pharmaceutiquement acceptable de celui-ci, éventuellement en association avec un véhicule pharmaceutiquement acceptable, comprenant au moins un autre composé utile pour la prévention ou le traitement d'une lésion photo-induite et/ou d'une dégénérescence de la rétine, notamment sélectionné dans le groupe constitué d'un anti-VEGF, d'un corticoïde et d'un antioxydant.

14. Produits contenant :
- au moins un composé de formule (I) tel que défini dans l'une des revendications 1 à 5, ou un sel pharmaceutiquement acceptable de celui-ci, et
- au moins un autre composé utile pour la prévention ou le traitement d'une lésion photo-induite et/ou d'une dégénérescence de la rétine, notamment sélectionné dans le groupe constitué d'un anti-VEGF, d'un corticoïde et d'un antioxydant,
comme produit de combinaison pour une utilisation dans la prévention ou le traitement d'une lésion photo-induite et/ou d'une dégénérescence de la rétine, et plus particulièrement dans la prévention ou le traitement de la photorétinite, de la rétinite pigmentaire, de la maculopathie liée à l'âge (MLA), ou de la dégénérescence maculaire liée à l'âge (DMLA), chez un individu, notamment un individu qui suit une photothérapie dynamique.

## Patentansprüche

1. Verbindung der folgenden Formel (I): 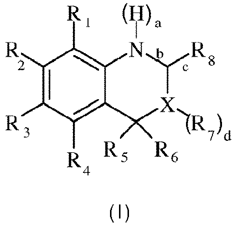 in der
- a für 0 oder 1 steht,
- b für eine Einfach- oder Doppelbindung steht,
- c für eine Einfach- oder Doppelbindung steht,
- d für 0 oder 1 steht,
- X für ein Sauerstoff- oder Stickstoffatom steht, mit der Einschränkung, dass im Fall, dass X ein Sauerstoffatom ist, d gleich 0 ist, und im Fall, dass X ein Stickstoffatom ist, d gleich 1 ist,
- R₁, R₂, R₃ und R₄, identisch oder verschieden, für ein Wasserstoffatom, ein Halogenatom, insbesondere unter F, Cl, Br und I ausgewählt, eine Hydroxygruppe oder eine Alkoxygruppe mit 1 oder 2 Kohlenstoffatomen steht,
- R₅ und R₆, identisch oder verschieden, für ein Wasserstoffatom, eine Alkyl- oder Zykloalkylgruppe mit 1 bis 6 Kohlenstoffatomen, oder eine Arylgruppe mit 6 Kohlenstoffatomen steht, deren aromatischer Kern gegebenenfalls mit einem oder mehreren Halogenatomen oder einer oder mehreren Hydroxy-, Alkoxygruppen mit 1 oder 2 Kohlenstoffatomen, Trifluormethyl- oder Nitrogruppen substituiert ist,
- R₇ für ein Wasserstoffatom, eine Hydroxygruppe oder eine Alkyl- oder Hydoxyalkylgruppe mit 1 bis 3 Kohlenstoffatomen steht,
- R₈ für ein Sauerstoff- oder eine -NR₉R₁₀-Gruppe steht, in der R₉ und R₁₀, identisch oder verschieden, für ein Wasserstoffatom, eine Hydroxygruppe oder eine Alkyl- oder Hydoxyalkylgruppe mit 1 bis 3 Kohlenstoffatomen, stehen, mit der Einschränkung, dass im Fall, das R₈ ein Sauerstoffatom ist, a gleich 1 ist, b eine Einfachbindung und c eine Doppelbindung ist, und dass, wenn R₈ für eine -NR⁹R₁₀-Gruppe steht, a gleich 0 ist, b eine Doppelbindung und c eine Einfachbindung ist,
oder ein pharmazeutisch verträgliches Salz davon,
zum Gebrauch in der Vorbeugung oder der Behandlung einer lichtinduzierten Läsion und/oder einer Degeneration der Retina eines Patienten.

2. Verbindung oder pharmazeutisch verträgliches Salz davon zum Gebrauch nach Patentanspruch 1, in der die Formel (I) durch die folgende Formel (II) vertreten wird: 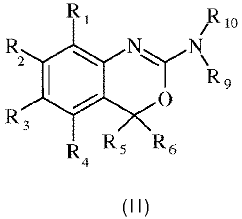 in der R₁, R₂, R₃, R₄, R₅, R₆, R₉, und R₁₀ wie oben definiert sind.

3. Verbindung oder pharmazeutisch verträgliches Salz davon zum Gebrauch nach Patentanspruch 1 oder 2 der folgenden Formel (IIIa), (IIIb) oder (IV):

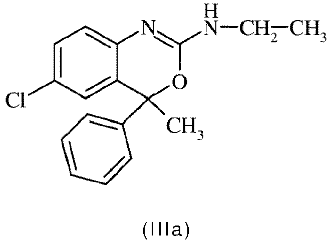

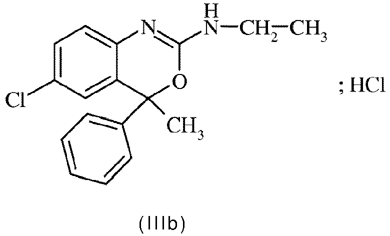

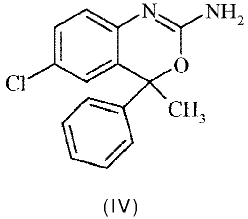

4. Verbindung oder pharmazeutisch verträgliches Salz davon zum Gebrauch nach Patentanspruch 1, in der die Formel (I) durch die folgende Formel (V) vertreten wird: 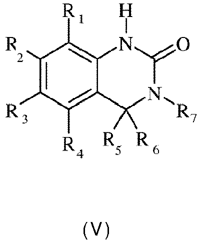 in der R₁, R₂, R₃, R₅, R₆ und R₇ wie im Patentanspruch 1 definiert sind.

5. Verbindung oder pharmazeutisch verträgliches Salz davon zum Gebrauch nach Patentanspruch 1 oder 4 der folgenden Formel (VI) oder (VII):

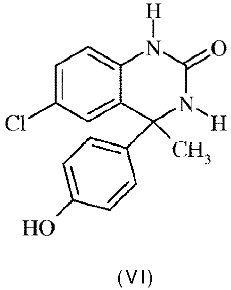

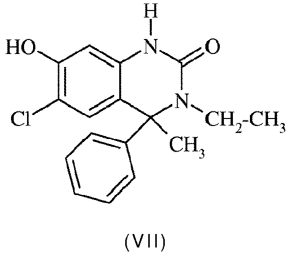

6. Verbindung oder pharmazeutisch verträgliches Salz davon zum Gebrauch nach einem der Patentansprüche 1 bis 5, in einer Einzeldosis von 50 mg bis 1500 mg abgepackt.

7. Verbindung oder pharmazeutisch verträgliches Salz davon zum Gebrauch nach einem der Patentansprüche 1 bis 6, in einer zur oralen Verabreichung geeigneten Form.

8. Verbindung oder pharmazeutisch verträgliches Salz davon zum Gebrauch nach einem der Patentansprüche 1 bis 7, in Form eines Pulvers, von Tabletten, Gelkapseln oder Tüten.

9. Verbindung oder pharmazeutisch verträgliches Salz davon zum Gebrauch nach einem der Patentansprüche 1 bis 8 zur Vorbeugung oder Behandlung von aktinischer Retinopathie, Retinopathia pigmentosa, altersbedingter Makulopathie oder altersbedingter Makuladegeneration.

10. Verbindung oder pharmazeutisch verträgliches Salz davon zum Gebrauch nach einem der Patentansprüche 1 bis 9 in Kombination mit mindestens einer anderen Verbindung, die zur Vorbeugung oder Behandlung einer lichtinduzierten Läsion und/oder einer Retinadegeneration nützlich ist, gewählt aus der Gruppe, bestehend aus einem Anti-VEGF-Medikament, einem Kortikoid und einem Antioxidans.

11. Verbindung oder pharmazeutisch verträgliches Salz davon zum Gebrauch nach einem der Patentansprüche 1 bis 10, in dem der Patient eine photodynamische Therapie erfährt.

12. Pharmazeutische Zubereitung, die als Wirkstoff mindestens eine Verbindung der Formel (I), wie sie in einem der Patentansprüche 1 bis 5 definiert ist, oder ein pharmazeutisch verträgliches Salz davon enthält,
gegebenenfalls in Verbindung mit einem pharmazeutisch verträglichen Vehikel, und optional mindestens eine andere Verbindung enthaltend, die zur Vorbeugung oder Behandlung einer lichtinduzierten Läsion und/oder einer Retinadegeneration nützlich ist, insbesondere gewählt aus der Gruppe, bestehend aus einem Anti-VEGF-Medikament, einem Kortikoid und einem Antioxidans
zum Gebrauch zur Vorbeugung oder Behandlung einer lichtinduzierten Läsion und/oder einer Retinadegeneration und insbesondere zur Vorbeugung oder Behandlung von aktinischer Retinopathie, Retinopathia pigmentosa, altersbedingter Makulopathie oder altersbedingter Makuladegeneration bei einem Patienten, insbesondere einem Patienten, der eine photodynamische Therapie erfährt.

13. Pharmazeutische Zubereitung, die als Wirkstoff mindestens eine Verbindung der Formel (I), wie sie in einem der Patentansprüche 1 bis 5 definiert ist, oder ein pharmazeutisch verträgliches Salz davon enthält, gegebenenfalls in Verbindung mit einem pharmazeutisch verträglichen Vehikel, und mindestens eine andere Verbindung enthaltend, die zur Vorbeugung oder Behandlung einer lichtinduzierten Läsion und/oder einer Retinadegeneration nützlich ist, insbesondere gewählt aus der Gruppe, bestehend aus einem Anti-VEGF-Medikament, einem Kortikoid und einem Antioxidans.

14. Produkte, enthaltend:
- mindestens eine Verbindung der Formel (I), wie sie in einem der Patentansprüche 1 bis 5 definiert ist, oder ein pharmazeutisch verträgliches Salz davon, und
- mindestens eine andere Verbindung, die zur Vorbeugung oder Behandlung einer lichtinduzierten Läsion und/oder einer Retinadegeneration nützlich ist, insbesondere gewählt aus der Gruppe, bestehend aus einem Anti-VEGF-Medikament, einem Kortikoid und einem Antioxidans,
als Kombinationspräparat zum Gebrauch zur Vorbeugung oder Behandlung einer lichtinduzierten Läsion und/oder einer Retinadegeneration und insbesondere zur Vorbeugung oder Behandlung von aktinischer Retinopathie, Retinopathia pigmentosa, altersbedingter Makulopathie oder altersbedingter Makuladegeneration bei einem Patienten, insbesondere einem Patienten, der eine photodynamische Therapie erfährt.

## Claims

1. A compound of the following formula (I): 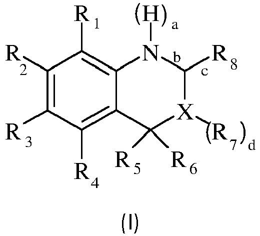 wherein:
- a represents 0 or 1;
- b represents a single bond or a double bond;
- c represents a single bond or a double bond;
- d represents 0 or 1;
- X represents an oxygen or nitrogen atom, provided that when X represents an oxygen atom then d has the value of 0 and when X represents a nitrogen atom then d has the value of 1;
- R₁, R₂, R₃, and R₄, which are the same or different, represent a hydrogen atom, a halogen atom, in particular selected from F, CI, Br, or I, a hydroxyl group, or an alkoxyl group having 1 or 2 carbon atoms;
- R₈ and R₆, which are the same or different, represent a hydrogen atom, an alkyl or cycloalkyl group having from 1 to 6 carbon atoms, or an aryl group having 6 carbon atoms in which the aromatic ring may be substituted by one or more halogen atoms or one or more hydroxyl groups, alkoxyl groups having 1 or 2 carbon atoms, trifluoromethyl groups or nitro groups;
- R₇ represents a hydrogen atom, a hydroxyl group, or an alkyl or hydroxyalkyl group having from 1 to 3 carbon atoms;
- R₈ represents an oxygen atom or a -NR₉R₁₀ group, R₉ and R₁₀, which are the same or different, representing a hydrogen atom, a hydroxyl group, or an alkyl or hydroxyalkyl group having from 1 to 3 carbon atoms, provided that when R₈ represents an oxygen atom then a has the value of 1, b represents a single bond and c represents a double bond and when R₈ represents a -NR₉R₁₀ group then a has the value of 0, b represents a double bond and c represents a single bond;
or pharmaceutically acceptable salt thereof,
for its use in the prevention or treatment of a light-induced damage and/or a degeneration of the retina in an individual.

2. The compound or pharmaceutically acceptable salt thereof for use according to claim 1, wherein formula (I) is represented by the following formula (II): 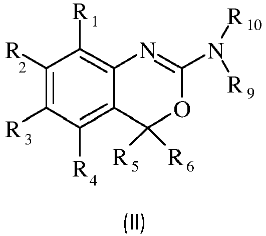 wherein R₁, R₂, R₃, R₄, R₅, R₆, R₉ and R₁₀ are as defined above.

3. The compound or pharmaceutically acceptable salt thereof for use according to claim 1 or 2, of the following formula (IIIa), (IIIb) or (IV):

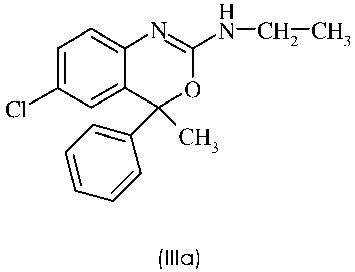

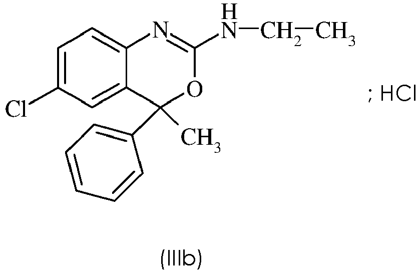

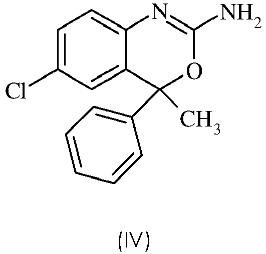

4. The compound or pharmaceutically acceptable salt thereof for use according to claim 1, wherein formula (I) is represented by the following formula (V): 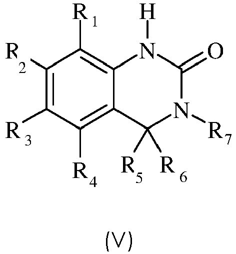 wherein R₁, R₂, R₃, R₅, R₆, and R₇ are as defined in claim 1.

5. The compound or pharmaceutically acceptable salt thereof for use according to claim 1 or 4, of the following formula (VI) or (VII):

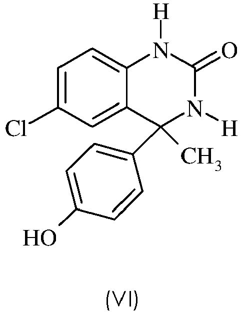

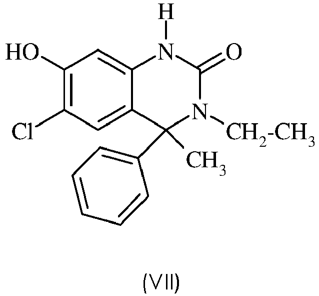

6. The compound or pharmaceutically acceptable salt thereof for use according to any one of claims 1 to 5, packaged in a unit dose of 50 mg to 1500 mg.

7. The compound or pharmaceutically acceptable salt thereof for use according to any one of claims 1 to 6, in a form suitable for administration by the oral route.

8. The compound or pharmaceutically acceptable salt thereof for use according to any one of claims 1 to 7, in the form of a powder, tablets, capsules or sachets.

9. The compound or pharmaceutically acceptable salt thereof for use according to any one of claims 1 to 8, in the prevention or treatment of photoretinitis, retinitis pigmentosa, age-related maculopathy (ARM), or age-related macular degeneration (AMD).

10. The compound or pharmaceutically acceptable salt thereof for use according to any one of claims 1 to 9, in combination with at least one additional compound useful for preventing or treating a light-induced damage and/or a degeneration of the retina selected from the group constituted of an anti-VEGF, a corticoid and an antioxidant.

11. The compound or pharmaceutically acceptable salt thereof for use according to any one of claims 1 to 10, wherein the individual is treated with dynamic phototherapy.

12. A pharmaceutical composition comprising as active ingredient at least one compound of formula (I) as defined in any of claims 1 to 5, or a pharmaceutically acceptable salt thereof,
optionally in association with a pharmaceutically acceptable vehicle, and optionally comprising at least one additional compound useful for the prevention or treatment of a light-induced damage and/or a degeneration of the retina, in particular selected from the group constituted of an anti-VEGF, a corticoid and an antioxidant,
for use in the prevention or treatment of a light-induced damage and/or a degeneration of the retina, and more particularly in the prevention or treatment of photoretinitis, retinitis pigmentosa, age-related maculopathy (ARM) or age-related macular degeneration (AMD), in an individual, in particular an individual treated with dynamic phototherapy.

13. A pharmaceutical composition comprising as active ingredient at least one compound of formula (I) as defined in any of claims 1 to 5, or a pharmaceutically acceptable salt thereof, optionally in association with a pharmaceutically acceptable vehicle, comprising at least one additional compound useful for the prevention or treatment of a light-induced damage and/or a degeneration of the retina, in particular selected from the group consisting of an anti-VEGF, a corticoid and an antioxidant.

14. Products containing:
- at least one compound of formula (I) as defined in any of claims 1 to 5, or a pharmaceutically acceptable salt thereof, and
- at least one additional compound useful for the prevention or treatment of a light-induced damage and/or a degeneration of the retina, in particular selected from the group consisting of an anti-VEGF, a corticoid and an antioxidant,
as a combined preparation for use in the prevention or treatment of a light-induced damage and/or a degeneration of the retina, and more particularly in the prevention or treatment of photoretinitis, retinitis pigmentosa, age-related maculopathy (ARM) or age-related macular degeneration (AMD), in an individual, in particular in an individual treated with dynamic phototherapy.
